# EUROPÄISCHE PATENTSCHRIFT

(11) **EP 1 426 401 B1**
(45) Veröffentlichungstag und Bekanntmachung des Hinweises auf die Patenterteilung: **14.05.2008**
(21) Anmeldenummer: 03026290.1
(22) Anmeldetag: 15.11.2003
(51) Int. Cl.: C08J 3/075, A61K 31/133

(54) **Emulgator-Wachs-Gele auf Wasserbasis**
Water-based emulgator wax gels
Gels à base d'eau comprenant un émulsifiant et une cire

(30) Priorität: 28.11.2002 DE 10255554
(43) Veröffentlichungstag der Anmeldung: 09.06.2004
(73) Patentinhaber: Evonik Goldschmidt GmbH, 45127 Essen (DE)
(72) Erfinder: Dietz, Thomas Dr., Virginia 23060 (US)

(56) Entgegenhaltungen:
- EP-A- 1 166 769
- WO-A-01/24767
- WO-A-98/53797
- US-A- 5 792 794
- US-A- 5 939 077

## Beschreibung

Die Erfindung betrifft Emulgator-Wachs-Gele auf Wasserbasis enthaltend hautidentische freie Sphingoidbasen ein Verfahren zu ihrer Herstellung, sowie ein Verfahren zur Herstellung von Öl-in-Wasser-Emulsionen unter Einsatz diesen Gele.

Seit einiger Zeit sind hautidentische Lipide wie Ceramide und Phytosphingosin kommerziell für den Einsatz in kosmetischen Produkten erhältlich. Hierbei handelt es sich um hochschmelzende, kristalline Substanzen mit nur geringer Löslichkeit in kosmetischen Ölen, was deren Verarbeitung und Stabilisierung in Endprodukten erheblich erschwert. So muss beispielsweise eine Ölphase, die 0,2 % Phytosphingosin enthält, auf über 90 °C erhitzt werden, damit das Phytosphingosin vollständig gelöst wird. 90 °C stellt jedoch für viele Produktionsanlagen ein Problem dar, da diese Temperatur nicht erreicht werden kann. Ein weiteres Problem, das auftreten kann, ist, dass während der Homogenisierung bei der Herstellung einer Öl-in-Wasser-Emulsion eine Inversion zu einer unbrauchbaren, inhomogenen Wasser-in-Öl-Emulsion stattfindet, wenn sich Phytosphingosin in der Ölphase befindet. Dieses Phänomen beobachtet man insbesondere bei eher hydrophoben O/W-Emulgatoren, den sogenannten Lipidemulgatoren.

WO-A-00/53568 beschreibt Sphingoidbasen-Derivate und deren Verwendung. Insbesondere werden Salze beschrieben, die eine wesentlich verbesserte Löslichkeit in wässriger Umgebung und damit eine verbesserte Effizienz in der topischen Anwendung aufweisen sollen. Aufgrund ihres Elektrolyt-Charakters stellen sie jedoch eine besondere Emulsionsbelastung dar und weisen Unverträglichkeit mit Stabilisatoren vom Hydrokolloid-Typ, wie beispielsweise Carbomer oder Xanthan Gum, auf.

WO-A-99/29293 beschreibt Zusammensetzungen, die eine Kombination einer freien Sphingoidbase und eines Ceramids enthalten. Die Zusammensetzungen sollen für die Anwendung auf der menschlichen Haut geeignet sein und dabei Barrierefunktionen aufweisen, insbesondere bei Hautbedingungen, die mit ungeordneter Deregulierung des Zellwachstums oder der Differenzierung der Entzündung oder eines infektiösen Stadiums belastet sind. So werden hier Öl-in-Wasser-Emulsionen beschrieben, die definierte Emulgatoren enthalten. Diese sollen geeignet sein, eine lamellare Phase (Flüssigkristallin- oder Gelphase) zu bilden. Die lamellaren Phasen werden an der Öl-Wasser-Grenzfläche einer Öl-in-Wasser-Emulsion gebildet und enthalten direkt die freie Sphingoidbase und das Ceramid.

WO-A-98/53797 betrifft eingekapselte, wasserunlösliche Wirkstoffe mit amphiphilem Charakter mit einem Gehalt an Wasser und mindestens einem Tensid aus der Gruppe der Ester von langkettigen Carbonsäuren mit Hydroxylgruppen enthaltenden Carbonsäuren oder deren Salzen und der Ester von langkettigen Carbonsäuren mit Polyalkoholen. Solche Wirkstoffe sollen bei der Zubereitung von pharmazeutischen, agrochemischen oder kosmetischen Formulierungen Verwendung finden. Ohne nähere Ausführungsbeispiele werden im Text als Wirkstoffe auch Ceramide, lipophile Amide aus einem gesättigten und ungesättigten Aminodiolrest (Diphytosphingosine und Sphingosine) und gesättigtem Aminotriolrest (Phytosphingosine), langkettigen Fettsäureresten und langkettigen Alkylresten im Aminodiolteil erwähnt. Der Wirkstoff wird gemeinsam mit dem Tensid auf 80 °C unter Rühren erhitzt und bei 75 bis 80 °C so lange vermischt, bis eine vollständig klare Lösung erzielt wird. Nach dem Abkühlen auf 25 °C (Raumtemperatur) wird das Gemisch auf etwa 40 °C erhitzt, Wasser hinzugefügt und intensiv gerührt, bis das System vollständig homogenisiert ist.

Aufgabe der Erfindung war es, eine Möglichkeit der Einarbeitung von hautidentischen freien Sphingoidbasen in Öl-in-Wasser-Emulsionen bereitzustellen, die die oben beschriebenen Probleme des Standes der Technik löst.

Die oben genannte Aufgabe wird erfindungsgemäß in einer ersten Ausführungsform gelöst durch Emulgator-Wachs-Gele auf Wasserbasis bestehend aus
a) 1 bis 4 Gew.-% hautidentischer freier Sphingoidbasen,
b) 0,5 bis 6,0 Gew.-% wenigstens eines nichtionischen oder anionischen Emulgators,
c) bis zu 8 Gew.-% wenigstens eines wachsartigen Konsistenzgebers,
d) 80 bis 98 Gew.-% Wasser und
e) 0 bis 10 Gew.-% weiterer Hilfsstoffe.

Es wurde überraschend gefunden, dass sich hautidentische freie Sphingoidbasen stabil in ein wässriges Emulgator-Wachs-Gel einarbeiten lassen. Dieses kann dann wiederum als Träger genutzt werden, das Lipid in eine Öl-in-Wasser-Emulsion einzubringen, indem man das Gel der Wasserphase vor der Emulgierung zusetzt. Es wurde außerdem gefunden, dass Emulsionen, in die auf diese Weise das Lipid eingearbeitet wurde, andere physikalische Eigenschaften wie beispielsweise Viskosität besitzen als Emulsionen, in denen das Lipid über die Ölphase eingearbeitet wurde, obwohl beide Emulsionen identische stoffliche Zusammensetzungen haben.

Ferner wurde gefunden, dass Emulsionen, die sich nicht herstellen lassen, wenn man das Lipid der Ölphase zusetzt, sich jedoch herstellen lassen, wenn man das Lipid über ein Emulgator-Wachs-Gel der Wasserphase zusetzt.

Besonders bevorzugt im Sinne der vorliegenden Erfindung sind freie hautidentische Sphingoidbasen, insbesondere Sphingosin, Sphinganin Phytosphingosin und 6-Hydroxy-4-Sphingenin.

Die erfindungsgemäßen Emulgator-Wachs-Gele auf Wasserbasis enthalten wenigstens einen oder auch gegebenenfalls mehrere Emulgatoren. Neben nichtionischen und anionischen Emulgatoren, können gegebenenfalls auch kationische oder zwitterionische Emulgatoren eingesetzt werden.

In einer bevorzugten Ausführungsform der vorliegenden Erfindung ist der Emulgator ausgewählt aus Fettsäuren und neutralisierten Fettsäuren; Glycerylmonofettsäureestern, ethoxylierten Fettalkoholen und/oder -Estern; hoch ethoxylierten Fettalkoholen und/oder niedrig ethoxylierten Fettalkoholen, Polyglycerinestern, Zuckerestern, Lecithinen und/oder Phospholipiden.

Als Emulgatoren kommen beispielsweise nichtionogene oder anionische Tenside aus mindestens einer der folgenden Gruppen in Frage:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- C_{12/18}-Fettsäuremono- und -diester von Anlagerungsprodukten von 1 bis 30 Mol Ethylenoxid an Glycerin;
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- Alkylmono- und -oligoglycoside mit 8 bis 22 Kohlenstoffatomen im Alkylrest und deren ethoxylierte Analoga;
- Anlagerungsprodukte von 15 bis 60 Mol Ethylenoxid an Rizinusöl und/oder gehärtetes Rizinusöl;
- Polyol- und insbesondere Polyglycerinester, wie beispielsweise Polyglycerinpolyricinoleat, Polyglycerin-12-hydroxystearat oder Polyglycerindimerat. Ebenfalls geeignet sind Gemische von Verbindungen aus mehreren dieser Substanzklassen;
- Anlagerungsprodukte von 2 bis 15 Mol Ethylenoxid an Rizinusöl und/oder gehärtetes Rizinusöl;
- Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (beispielsweise Sorbit), Alkylglucoside (beispielsweise Methylglucosid, Butylglucosid, Laurylglucosid) sowie Polyglucoside (beispielsweise Cellulose);
- Mono-, Di- und Trialkylphosphate sowie Mono-, Di-und/oder Tri-PEG-alkylphosphate;
- Wollwachsalkohole;
- Polysiloxan-Polyether-Copolymere und entsprechende Derivate;
- Mischester aus Pentaerythrit, Fettsäuren, Citronensäure und Fettalkohol gemäß DE-B-11 65 574 und/oder Mischester von Fettsäuren mit 6 bis 22 Kohlenstoffatomen, Methylglucose und Polyolen, vorzugsweise Glycerin oder Polyglycerin sowie
- Polyalkylenglycole.
   Die Anlagerungsprodukte von Ethylenoxid und/oder von Propylenoxid an Fettalkohole, Fettsäuren, Alkylphenole, Glycerinmono- und diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.
   Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- oder eine Sulfonatgruppe tragen. Besonders geeignete zwitterionische Tenside sind die sogenannten Betaine, wie die N-Alkyl-N,N-dimethylammoniumglycinate, beispielsweise das Cocosalkyldimethylammoniumglycinat, N-Acylaminopropyl-N,N-dimethylammoniumglycinate, beispielsweise das Cocosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Cocosacylaminoethylhydroxyethylcarboxymethylglycinat.
   Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Cocosalkylaminopropionat, das Cocosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.
   Neben den Sphingolipiden, Emulgatoren und Wasser enthalten die erfindungsgemäßen Gele notwendigerweise wenigstens einen oder gegebenenfalls auch mehrere Konsistenzgeber.

Als Konsistenzgeber kommen in erster Linie Fettalkohole oder Hydroxyfettalkholole mit 12 bis 22 und vorzugsweise 16 bis 18 Kohlenstoffatomen und daneben Partialglyceride, Fettsäuren oder Hydroxyfettsäuren in Betracht.

Die Menge des eingesetzten Konsistenzgebers richtet sich im Wesentlichen nach der gewünschten Viskosität des herzustellenden Gels. Dementsprechend ist es im Sinne der vorliegenden Erfindung bevorzugt, wenn die Gele den Konsistenzgeber in einer Menge von 1 bis 8 Gew.-% enthalten.

Wenn im Sinne der vorliegenden Erfindung der Einsatz von wachsartigen Konsistenzgebern definiert ist, so werden hierunter bei 20 °C knetbare feste bis brüchig harte, grob bis feinkristalline, durchscheinende bis opake, jedoch nicht glasartige; über 40 °C ohne Zersetzung schmelzende, schon wenig oberhalb des Schmelzpunkts verhältnismäßig niedrig viskose und nicht fadenziehende, stark temperaturabhängige Konsistenz und Löslichkeit, unter leichtem Druck polierbare Konsistenzgeber verstanden, die sich von ähnlichen synthetischen oder natürlichen Produkten, beispielsweise Harzen, plastischen Massen, Metallseifen usw. hauptsächlich davon unterscheiden, dass sie in der Regel etwa zwischen 50 und 90 °C, in Ausnahmefällen auch bis etwa 200 °C in den schmelzflüssigen, niedrigviskosen Zustand übergehen und praktisch frei von aschebildenden Verbindungen sind.

Die Menge an Wasser, die in den erfindungsgemäßen Gelen vorhanden sein kann, kann in weiten Bereichen variiert werden. Im Sinne der vorliegenden Erfindung sind Gele, die Wasser in einer Menge von 80 bis 98 Gew.-% enthalten, einsetzbar.

Die erfindungsgemäßen Gele können gegebenenfalls weitere Hilfsstoffe enthalten, die auf dem Gebiet der Kosmetik oder Pharmazie üblich sind. Besonders bevorzugt im Sinne der vorliegenden Erfindung sind Feuchthaltemittel und/oder Konservierungsmittel. Entsprechende Hilfsstoffe können erfindungsgemäß in den Gelen in einer Menge von 0,01 bis 10,0 Gew.-% enthalten sein.

Eine weitere Ausführungsform der vorliegenden Erfindung besteht in der Herstellung der Emulgator-Wachs-Gele, wie sie eingangs definiert sind. Erfindungsgemäß können die Emulgator-Wachs-Gele dadurch hergestellt werden, dass man hautidentische Sphingolipide mit wenigstens einem Emulgator, wenigstens einem Konsistenzgeber und gegebenenfalls weiteren Hilfsstoffen aufschmilzt und bei gegenüber Raumtemperatur erhöhter Temperatur mit Wasser in Kontakt bringt, homogenisiert und anschließend unter Rühren auf Raumtemperatur abkühlt.

Die so erhältlichen Emulgator-Wachs-Gele sind besonders zur Herstellung von Öl-in-Wasser-Emulsionen geeignet, die Sphingolipide gegebenenfalls auch in hohen Mengenanteilen enthalten.

Besonders bevorzugt im Sinne der vorliegenden Erfindung wird das Emulgator-Wachs-Gel in die wässrige Phase der Emulsion eingearbeitet.

Die so enhältlichen Öl-in-Wasser-Emulsionen, enthalten freie Sphingoidbasen gegebenenfalls in einer sehr hohen und damit effizienten Konzentration, beispielsweise in einer Menge von 0,01 bis 0,4 Gew.-%.

### Beispiele:

### Beispiel 1:

### Emulgator-Wachs-Gel mit Phytosphingosin:

Die Komponenten von Phase A wurden auf 105 bis 110 °C erhitzt, bis das Phytosphingosin klar gelöst war. Anschließend wurden die Komponenten von Phase B auf 90 °C erhitzt, zur 95 °C heißen Phase A gegeben und intensiv homogenisiert. Es wurde dann unter mäßigem Rühren auf Raumtemperatur abgekühlt.

| | | |
|---|---|---|
| **A** | Ceteareth-25 | 2,0 % |
| | Glycerylstearat | 2,5 % |
| | Cetearylalkohol | 3,5 % |
| | Stearinsäure | 1,0 % |
| | Phytosphingosin | 2,0 % |
| | | |
| **B** | Glycerin | 3,0 % |
| | Benzylalkohol, Methylchloroisothiazolinon, Methylisothiazolinon | 0,1 % |
| | Wasser | 85,9 % |

### Vergleichsbeispiel 1:

Emulsion, die sich nicht herstellen lässt, wenn man Phytosphingosin der Ölphase zusetzt:

Die Komponenten der Phase A wurden auf 90 °C erhitzt, bis das Phytosphingosin vollständig gelöst war. Die Komponenten von Phase B wurden auf 90 °C erhitzt, zur Phase A gegeben und homogenisiert. Während des Homogenisierens schlug die anfänglich dünnflüssige O/W-Emulsion in eine dickflüssige und inhomogene W/O-Emulsion um, die nicht mehr verwendet werden konnte.

| | | |
|---|---|---|
| **A** | Polyglyceryl-3-Methylgludose-Disteareat | 3,0 % |
| | Cetearylalkohol | 1,75 % |
| | Glycerylstearat | 0,75 % |
| | Isocetylpalmitat | 5,5 % |
| | Ethylhexylstearat | 9,0 % |
| | Avocadoöl | 3,0 % |
| | Tocopherylacetat | 1,0 % |
| | Phytosphingosin | 0,2 % |
| | | |
| **B** | Glycerin | 3,0 % |
| | Allantoin | 0,1 % |
| | Chloracetamid und Natriumbenzoat | 0,1 % |
| | Wasser | 72,6 % |

### Beispiel 2:

Emulsion, die sich mit dem wässrigen Emulgator-Wachs-Gel aus Beispiel 1 herstellen lässt.

Die Komponenten der Phase A wurden auf 75 °C erhitzt. Die Komponenten von Phase B wurden auf 75 °C erhitzt, zur Phase A gegeben und homogenisiert. Anschließend wurde unter mäßigem Rühren auf Raumtemperatur abgekühlt. Die entstandene Emulsion hatte ein glattes und brillantes Aussehen und wies eine sehr gute Lagerstabilität auf.

| | | |
|---|---|---|
| **A** | Polyglyceryl-3-Methylglucose-Disteareat | 3,0 % |
| | Cetearylalkohol | 1,75 % |
| | Glycerylstearat | 0,75 % |
| | Isocetylpalmitat | 5,5 % |
| | Ethylhexylstearat | 9,0 % |
| | Avocadoöl | 3,0 % |
| | Tocopherylacetat | 1,0 % |
| | | |
| **B** | Glycerin | 3,0 % |
| | Allantoin | 0,1 % |
| | Emulgator-Wachs-Gel mit Phytosphingosin (2 %) aus Beispiel 1 | 10,0 % |
| | Chloracetamid und Natriumbenzoat | 0,1 % |
| | Wasser | 62,6 % |

### Beispiel 3/Vergleichsbeispiel 2:

Emulsionen mit identischer Zusammensetzung, jedoch unterschiedlichen physikalischen Eigenschaften in Abhängigkeit der Einarbeitung von Phytosphingosin:

### Herstellung von Vergleichsbeispiel 2:

Die Komponenten der Phase A wurden auf 90 °C erhitzt, bis das Phytosphingosin vollständig gelöst war. Die Komponenten von Phase B wurden auf 90 °C erhitzt, zur Phase A gegeben und homogenisiert. Anschließend wurde unter leichtem Rühren auf 65 °C abgekühlt, Phase C und D zugegeben, erneut kurz homogenisiert und weiter unter leichtem Rühren auf Raumtemperatur abgekühlt.

| | | |
|---|---|---|
| **A** | Bis-PEG/PPG-16/16 PEG/PPG-16/16 Dimethicon (und) Capryl/Caprin Triglycerid | 1,0 % |
| | Ceteareth-25 | 1,0 % |
| | Glycerylstearat | 4,0 % |
| | Cetearylalkohol | 1,5 % |
| | Stearinsäure | 0,5 % |
| | Decylcocoat | 5,3 % |
| | Ethylhexylpalmitat | 5,0 % |
| | Capryl/Caprin Triglycerid | 6,5 % |
| | Phytosphingosin | 0,2 % |
| | | |
| **B** | Glycerin | 3,0 % |
| | Chloracetamid (und) Natriumbenzoat | 0,1 % |
| | Wasser | 70,72 % |
| **C** | Natriumhydroxid | 0,43 % |
| **D** | Carbomer | 0,15 % |
| | Ethylhexylpalmitat | 0,6 % |

| | | |
|---|---|---|
| Viskosität (Brookfield, Spindel C, 10 UpM): 70 Pas. | | |

### Herstellung von Beispiel 3:

Die Komponenten der Phase A wurden auf 75 °C erhitzt. Die Komponenten der Phase B wurden auf 75 °C erhitzt, zur Phase A gegeben und homogenisiert. Anschließend wurde unter leichtem Rühren auf 65 °C abgekühlt, Phase C und D zugegeben, erneut homogenisiert und weiter unter leichtem Rühren auf Raumtemperatur abgekühlt.

| | | |
|---|---|---|
| **A** | Bis-PEG/PPG-16/16 PEG/PPG-16/16 Dimethicon (und) Capryl/Caprin Triglycerid | 1,0 % |
| | Ceteareth-25 | 0,8 % |
| | Glycerylstearat | 3,75 % |
| | Cetearylalkohol | 1,15 % |
| | Stearinsäure | 0,4 % |
| | Decylcocoate | 5,3 % |
| | Ethylhexylpalmitat | 5,0 % |
| | Capryl/Caprin Triglycerid | 6,5 % |
| **B** | Glycerin | 2,7 % |
| | Chloracetamid (und) Natriumbenzoat | 0,1 % |
| | Wasser | 62,12 % |
| | Emulgator-Wachs-Gel mit Phytosphingosin (2 %) aus Beispiel 1 | 10,0 % |
| **C** | Natriumhydroxid | 0,43 % |
| **D** | Carbomer | 0,15 % |
| | Ethylhexylpalmitat | 0,6 % |

| | | |
|---|---|---|
| Viskosität (Brookfield, Spindel C, 10 UpM): > 100 Pas | | |

## Patentansprüche

1. Emulgator-Wachs-Gele auf Wasserbasis bestehend aus
a) 1 bis 4 Gew.-% hautidentischer freier Sphingoidbasen,
b) 0,5 bis 6,0 Gew.-% wenigstens eines nichtionischen oder anionischen Emulgators,
c) bis zu 8 Gew.-% wenigstens eines wachsartigen Konsistenzgebers,
d) 80 bis 98 Gew.-% Wasser und
e) 0 bis 10 Gew.-% weiterer Hilfsstoffe.

2. Gele nach Anspruch 1, **dadurch gekennzeichnet, dass** die freien Sphingoidbasen Sphingosin, Sphinganin, Phytosphingosin und 6-Hydroxy-4-Sphingenin sind.

3. Gele nach einem der Ansprüche 1 und 2, **dadurch gekennzeichnet, dass** der Emulgator ausgewählt ist aus Fettsäuren und neutralisierten Fettsäuren; Glyceryl-mono-Fettsäureestern, ethoxylierten Fettalkoholen und/oder -Estern; Polyglycerinestern, Zuckerestern, Lecithinen und/oder Phospholipiden.

4. Gele nach einem der Ansprüche 1 bis 3, **dadurch gekennzeichnet, dass** der Konsistenzgeber ausgewählt ist aus Fettalkoholen oder Hydroxyfettalkoholen mit 12 bis 22, insbesondere 16 bis 18 Kohlenstoffatomen, Partialglyceriden, Fettsäuren und Hydroxyfettsäuren.

5. Gele nach einem der Ansprüche 1 bis 4, **dadurch gekennzeichnet, dass** die Hilfsstoffe ausgewählt sind aus Feuchthaltemitteln und/oder Konservierungsmitteln.

6. Verfahren zur Herstellung von Emulgator-Wachs-Gelen nach einem der Ansprüche 1 bis 5, **dadurch gekennzeichnet, dass** man hautidentische freie Sphingoidbasen mit wenigstens einem Emulgator, wenigstens einem Konsistenzgeber und gegebenenfalls weiteren Hilfsstoffen aufschmilzt und bei gegenüber Raumtemperatur erhöhter Temperatur mit Wasser in Kontakt bringt, homogenisiert und anschließend unter Rühren auf Raumtemperatur abkühlt.

7. Verfahren zur Herstellung von Öl-in-Wasser-Emulsionen, die freie Sphingoidbasen enthalten, **dadurch gekennzeichnet, dass** man das Emulgator-Wachs-Gel gemäß den Ansprüchen 1 bis 5 in die wässrige Phase der Emulsion einarbeitet.

8. Verfahren nach Anspruch 7, **dadurch gekennzeichnet, dass** man das Emulgator-Wachs-Gel in einer Menge einsetzt, dass die Öl-in-Wasser-Emulsion freie Sphingoidbasen in einer Menge von 0,01 bis 0,4 Gew.-% enthält.

## Claims

1. Water-based emulsifier wax gels consisting of
a) 1 to 4% by weight of skin-identical free sphingoid bases,
b) 0.5 to 6.0% by weight of at least one nonionic or anionic emulsifier,
c) up to 8% by weight of at least one wax-like consistency regulator,
d) 80 to 98% by weight of water and
e) 0 to 10% by weight of further auxiliaries.

2. Gels according to Claim 1, **characterized in that** the free sphingoid bases are sphingosine, sphinganine, phytosphingosine and 6-hydroxy-4-sphingenine.

3. Gels according to one of Claims 1 and 2, **characterized in that** the emulsifier is selected from fatty acids and neutralized fatty acids; glycerol mono-fatty acid esters, ethoxylated fatty alcohols and/or fatty esters; polyglycerol esters, sugar esters, lecithins and/or phospholipids.

4. Gels according to one of Claims 1 to 3, **characterized in that** the consistency regulator is selected from the fatty alcohols or hydroxy fatty alcohols having 12 to 22, in particular 16 to 18, carbon atoms, partial glycerides, fatty acids and hydroxy fatty acids.

5. Gels according to one of Claims 1 to 4, **characterized in that** the auxiliaries are selected from humectants and/or preservatives.

6. Process for the preparation of emulsifier wax gels according to one of Claims 1 to 5, **characterized in that** skin-identical free sphingoid bases are melted with at least one emulsifier, at least one consistency regulator and, optionally, further auxiliaries and, at a temperature higher than room temperature, brought into contact with water, homogenized and then cooled to room temperature with stirring.

7. Process for the preparation of oil-in-water emulsions which comprise free sphingoid bases, **characterized in that** the emulsifier wax gel according to Claims 1 to 5, is incorporated into the aqueous phase of the emulsion.

8. Process according to Claim 7, **characterized in that** the emulsifier wax gel is used in an amount such that the oil-in-water emulsion comprises free sphingoid bases in an amount of from 0.01 to 0.4% by weight.

## Revendications

1. Gels à base aqueuse comprenant un émulsifiant et une cire, constitués de
a) 1 à 4 % en poids de bases sphingoïdes libres, identiques à celles de la peau,
b) 0,5 à 6,0 % en poids d'au moins un émulsifiant anionique ou non ionique,
c) jusqu'à 8 % en poids d'au moins un agent de consistance cireux,
d) 80 à 98 % en poids d'eau et
e) 0 à 10 % en poids d'autres adjuvants.

2. Gels selon la revendication 1, **caractérisés en ce que** les bases sphingoïdes libres sont la sphingosine, la sphinganine, la phytosphingosine et la 6-hydro-4-sphingénine.

3. Gels selon la revendication 1 ou 2, **caractérisés en ce que** l'émulsifiant est choisi parmi des acides gras et des acides gras neutralisés ; des monoesters de glycéryle avec des acides gras, des alcools gras et/ou esters gras éthoxylés; des esters de polyglycérol, des esters de sucres, des lécithines et/ou des phospholipides.

4. Gels selon l'une quelconque des revendications 1 à 3, **caractérisés en ce que** l'agent de consistance est choisi parmi les alcools gras ou alcools gras hydroxylés ayant de 12 à 22, en particulier de 16 à 18 atomes de carbone, des glycérides partiels, des acides gras et des acides gras hydroxylés.

5. Gels selon l'une quelconque des revendications 1 à 4, **caractérisés en ce que** les adjuvants sont choisis parmi les agents hydratants et/ou les conservateurs.

6. Procédé pour la préparation de gels comprenant un émulsifiant et une cire, selon l'une quelconque des revendications 1 à 5, **caractérisé en ce qu'**on fait fondre des bases sphingoïdes libres, identiques à celles de la peau, avec au moins un émulsifiant, au moins un agent de consistance et éventuellement d'autres adjuvants, et on les met en contact avec de l'eau à une température élevée par rapport à la température ambiante, on homogénéise et ensuite on refroidit jusqu'à la température ambiante, sous agitation.

7. Procédé pour la préparation d'émulsions huile-dans eau qui contiennent des bases sphingoïdes libres, **caractérisé en ce que** le gel comprenant un émulsifient et une cire selon les revendications 1 à 5 est incorporé dans la phase aqueuse de l'émulsion.

8. Procédé selon la revendication 7, **caractérisé en ce que** le gel comprenant un émulsifiant et une cire est utilisé en une quantité telle que l'émulsion huile-dans-eau contienne des bases sphingoïdes libres en une quantité de 0,01 à 0,4 % en poids.
